# EUROPEAN PATENT APPLICATION

(11) **EP 4 640 182 A1**
(43) Date of publication of application: **29.10.2025**
(21) Application number: 24893245.1
(22) Date of filing: 06.11.2024
(51) Int. Cl.: A61C 17/34, A61C 17/20, A61C 17/22, A61C 19/06, A61N 5/06, A61B 1/24, A61B 1/04

(54) **ELECTRIC TOOTHBRUSH HEAD**

(30) Priority: 20.11.2023 CN 202311549720
(71) Applicant: Bixdo (SH) Healthcare Technology Co., Ltd., Shanghai 201100 (CN); Guangdong Bixdo Health Technology Co., Ltd., Foshan, Guangdong 528311 (CN)
(72) Inventor: HAN, Zhongping, Shanghai 201100 (CN); TAN, Chengshen, Shanghai 201100 (CN); QIAN, Dongdong, Shanghai 201100 (CN); WANG, Xuefeng, Shanghai 201100 (CN); PENG, Jun, Shanghai 201100 (CN)
(74) Representative: karo IP
(86) International application number: PCT/CN2024/130347
(87) International publication number: WO 2025/108087

(57) **Abstract**

The present application provides an electric toothbrush head and belongs to the field of electric toothbrushes. The electric toothbrush head includes a brush head body, an power-consuming component, and a shaft linkage member. The power-consuming component is disposed within the brush head body. The shaft linkage member is disposed within the brush head body and is configured to be in linkage connection with a motor shaft of a motor in the electric toothbrush. The shaft linkage member is provided with a first electrical connector and a second electrical connector. One end of the first electrical connector is conductively connected to the power-consuming component, and the other end of the first electrical connector is conductively connected to a power supply component of the electric toothbrush. One end of the second electrical connector is conductively connected to the power-consuming component, and the other end of the second electrical connector is conductively connected to the power supply component of the electric toothbrush. In this embodiment, the first electrical connector and the second electrical connector are arranged on the shaft linkage member. In the structure aspect, the shaft linkage member is installed and connected to the brush head body; and in the circuit aspect, the two electrical connectors are conductively connected to a positive electrode and a negative electrode of the power-consuming component inside the brush head body. In this way, it achieves stable assembly in the structure aspect and convenient electrical conduction in the circuit aspect.

## Description

### TECHNICAL FIELD

The present disclosure relates to the field of electric toothbrushes, and particularly provides an electric toothbrush head.

### BACKGROUND

With the development of social intelligence, more and more electrical appliances have appeared in people's lives. In addition, with the improvement of living standards, more and more consumers pay attention to oral health issues. For example, an electric toothbrush as a type of electrical appliances, utilizes a sonic motor as a driver to generate power, thereby creating high-frequency reciprocating vibrations with a certain amplitude so as to drive the brush head for high-frequency swing, and thus achieving teeth cleaning and greatly facilitating daily life. At present, there are relatively few electric toothbrushes on the market in which the brush head is luminous and has additional functions, most electric toothbrushes are conventional electric toothbrushes. A few blue or red light electric toothbrushes often require a significant change to a motor output shaft to complete the power supply wiring so as to supply power to the brush heads, which makes the structures complex. Alternatively, due to the increase in number of components or the change in motor type, the motion transmission efficiency may suffer a significant loss, causing the swing amplitude and frequency of brush heads to be reduced. For example, a Chinese Patent CN215019476U discloses a structure of a sonic electric toothbrush with sterilization, disinfection and blue light whitening functions, and the sonic electric toothbrush accomplishes power supply through wireless charging coils, causing an increase in the number of components and resulting in an unsatisfactory effect of the brush head swing. Alternatively, in order to accommodate conductive devices, a volume of the brush head and the motor output shaft is increased, thereby reducing the user experience. Based above, the present disclosure has been developed.

### SUMMARY

In view of the above, according to one aspect, the present disclosure aims to solve the problem that the power supply structure of the sonic motor in the electric toothbrushes on the market is relatively complex, a significant change to the motor output shaft is required and would affect the motion transmission efficiency.

According to another aspect, the present disclosure aims to solve the problem that the power supply structure of the sonic motor in some electric toothbrushes in the market has a complex wiring arrangement, causing assembling and mounting can not be achieved.

According to yet another aspect, the present disclosure aims to solve the problem that in some electric toothbrushes in the market, the sonic motor requires change in the spatial structure of the motor output shaft or brush head to accommodate the conductive components.

To address one of these problems, the present disclosure provides an electric toothbrush brush head. The electric toothbrush brush head includes a brush head body, a power-consuming component, and a shaft linkage member. The power-consuming component is disposed within the brush head body. The shaft linkage member is disposed within the brush head body and is configured to be in linkage connection with a motor shaft of a motor in the electric toothbrush. The shaft linkage member is provided with a first electrical connector and a second electrical connector, the first electrical connector has one end that is conductively connected to the power-consuming component and another end that is conductively connected to a power supply component of the electric toothbrush. The second electrical connector has one end that is conductively connected to the power-consuming component and another end that is conductively connected to the power supply component of the electric toothbrush.

Preferably, the brush head body includes a bristle assembly and a brush head handle. The bristle assembly is mounted to the brush head handle, and the power-consuming component is positioned at a location corresponding to the bristle assembly. The brush head handle has a first insertion cavity, the shaft linkage member is configured to be inserted into the first insertion cavity and to be linked to the brush head handle. The shaft linkage member has a second insertion cavity, the motor shaft is configured to be inserted into the second insertion cavity and to be linked to the shaft linkage member.

Preferably, the shaft linkage member is circumferentially restrained relative to the brush head handle through a first circumferential limiting mechanism, and the shaft linkage member is axially restrained relative to the brush head handle through a first axial limiting mechanism.

Preferably, the first circumferential limiting mechanism includes a first circumferential limiting rib and a first circumferential limiting groove. The first circumferential limiting rib and the first circumferential limiting groove are respectively disposed on an inner wall of the first insertion cavity and an outer wall of the shaft linkage member.

Preferably, the first axial limiting mechanism includes a first axial limiting rib and a first axial limiting groove. The first axial limiting rib and the first axial limiting groove are respectively disposed on an outer wall of the shaft linkage member and an inner wall of the first insertion cavity.

Preferably, the first axial limiting groove is a first ring groove, the first ring groove is disposed on the inner wall of the first insertion cavity that is close to an opening. The first axial limiting rib is a first ring rib, and the first ring rib is disposed on the outer wall of the shaft linkage member and is capable of being interference-fitted into the first ring groove.

Preferably, the motor shaft is inserted into the second insertion cavity, the motor shaft is circumferentially restrained relative to the shaft linkage member through the second circumferential limiting mechanism, and the motor shaft is axially restrained relative to the shaft linkage member through the second axial limiting mechanism.

Preferably, the second circumferential limiting mechanism includes a second circumferential limiting step surface and a second circumferential limiting rib. The second circumferential limiting step surface is formed on the motor shaft, the second circumferential limiting rib is disposed on an inner wall of the second insertion cavity, and the second circumferential limiting rib presses against the second circumferential limiting step surface so as to restrict a relative circumferential rotation.

Preferably, the second axial limiting mechanism includes a second axial limiting slot and a second axial limiting snap-fit. The second axial limiting slot is formed on the motor shaft, and the second axial limiting snap-fit is disposed on the shaft linkage member so as to achieve an axial position limiting relative to the second axial limiting slot.

Preferably, the one end of the first electrical connector and the one end of the second electrical connector are respectively connected to a positive electrode and a negative electrode of the power-consuming component via wires. The other end of the first electrical connector and the other end of the second electrical connector are respectively connected to a positive electrode and a negative electrode of the power supply component via wires.

Preferably, the shaft linkage member has a first mounting port at a side thereof, and has a second mounting port at a top thereof. The motor is a sonic motor, and the motor shaft is a hollow conductive motor shaft. The hollow conductive motor shaft is provided with a first electrical terminal at a position corresponding to the first mounting port and a second electrical terminal at a position corresponding to the second mounting port. The first electrical terminal and the second electrical terminal are insulated from each other. The first electrical connector is mounted at the first mounting port and is in electrical contact with the first electrical terminal. The first electrical terminal is connected to the power supply component via a first wire. The second electrical connector is mounted at the second mounting port and is in electrical contact with the second electrical terminal. The second electrical terminal is connected to the power supply component via a second wire passing through a hollow inner cavity of the hollow conductive motor shaft.

Preferably, the electric toothbrush head further includes a conductive assembly. The conductive assembly includes an electrode and an insulating mounting base. The electrode is defined as the second electrical terminal. The electrode is mounted at the second mounting port via the insulating mounting base. One end of the second wire is connected to the electrode, and another end of the second wire passes through the hollow conductive motor shaft and is connected to the power supply component.

Preferably, the second electrical connector is a conductive probe, the conductive probe is inserted into the second mounting port and is in electrical contact with the electrode.

Preferably, the first electrical connector is a conductive elastic plate, the conductive elastic plate has an elastic deformation portion at a bottom thereof. The shaft linkage member is provided with a guide slot along an insertion direction. The first mounting port is located in the guide slot. The first electrical terminal is defined as an outer surface of the motor shaft at a position corresponding to the first mounting port. The conductive elastic plate compresses the elastic deformation portion so as to be inserted along the guide slot into the first mounting port, the elastic deformation portion extends into the first mounting port and is in electrical contact with the first electrical terminal.

Preferably, the power-consuming component includes a light board and multiple light beads, and the multiple light beads are mounted on the light board. The light bead is implemented as one of a blue light whitening bead, a red light physiotherapy bead, or a purple light sterilization bead or any combination thereof.

Preferably, the bristle assembly includes a bristle seat and multiple bristle tufts planted on the bristle seat. The bristle tuft is formed by clustering transparent light-guiding brush filaments, and the bristle tuft is positioned at a location corresponding to the light bead.

Preferably, the power-consuming component is implemented as a sensor and/or a photographic component.

The beneficial effects of the above technical solutions may be resulted from any one of the following items or any combination thereof
The first electrical connector and the second electrical connector are arranged on the shaft linkage member. In the structure aspect, the shaft linkage member is installed and connected to the brush head body; and in the circuit aspect, the two electrical connectors are conductively connected to a positive electrode and a negative electrode of the power-consuming component inside the brush head body. In this way, it achieves stable assembly in the structure aspect and convenient electrical conduction in the circuit aspect. The two electrical connectors are stably connected, through the shaft linkage member, to the positive electrode and the negative electrode of the power-consuming component on the one hand, and are conveniently conductively connected to a positive electrode and a negative electrode of the power supply component on the other hand. The overall structure uses the shaft linkage member as a carrier, and there is no need for extra structural design, therefore no damage is made to the layout of the components of the electric toothbrush, and the original space occupancy rate of the brush head and the motor shaft will not be reduced.

In the present disclosure, the motor shaft of the sonic motor is implemented as a hollow conductive structure. An interior of the motor shaft is implemented as a wiring channel. The conductive assembly passes through and out of the motor shaft along an axis of the motor shaft for connecting to an element, so as to form a first conducting path. The motor shaft itself can be used as a second conducting path due to its conductive property. Both ends of each of the two conducting paths are connected to the power-consuming component and the power supply respectively, so as to form an electrically conductive circuit. The characteristics of the structure of the sonic motor itself is fully utilized. Only the motor shaft needs to be set to be hollow, and there is no need for a significant structural modification to the sonic motor. Moreover, the two electrical terminals are integrated with the motor shaft itself, thus the back-and-forth vibrations of the motor at a high frequency does not affect the electrical connection efficiency and no extra wiring layout is required. The two electrical connectors can be contact with corresponding inner parts stably via the shaft linkage member and can be mounted with an external part in an assembled way, and thus has incomparable advantages in both security and convenience.

The external output linkage is completed through the shaft linkage member. At the same time, due to a structure of the shaft linkage member, the shaft linkage member can be installed on the brush head body and the brush head body can be assembled with the mounting ports on the shaft linkage member, so that electrical connection of the two electrical connectors can be achieved.

Wire paths can be completely routed inside the hollow conductive motor shaft, or one wire path can be led out from the hollow conductive motor shaft via a wire to connect to an external element. In this way, the present application has a strong adaptability and a wide range of application. It is particularly suitable for a conductive structure in which the power supply is located on one side of the sonic motor and the power-consuming component is located on the other side of the sonic motor. It has a strong integration, a high space utilization and low difficulty of arranging wires, and improves the durability.

The two electrical connectors are respectively connected to the two electrical terminals in an assembled way, and the assembling is very convenient. The conducting paths are clear. The conducting path of the power supply component is integrated on the sonic motor and the shaft linkage member, and the conducting path of the power-consuming component is integrated into brush head body. The conducting path of the power supply component and the conducting path of the power-consuming component only need to be assembled and installed to contact with each other. The shaft linkage member further stabilizes the contact structure between two conducting paths. Compared with the pure wire connection available on the market, reliability in the present application is greatly improved.

The shaft linkage member is inserted into the brush head handle in the electric toothbrush, so that the sonic motor electromagnetically drives the hollow conductive motor shaft and thus the hollow conductive motor shaft drives the brush head handle to rotate reciprocally at a high-frequency through the shaft linkage member. Finally, the brush head body is driven to swing at a high frequency. The two electrical connectors are implemented as the conductive probe and conductive elastic plate respectively. The conductive probe is inserted into the first electrode. The conductive elastic plate is assembled with the shaft linkage member via the guide slot. The structure of the shaft linkage member achieves stable electrical contact, thereby achieving a high assembly performance and a good stability.

The power-consuming component is implemented as a light board and a light bead with a light effect function, and can be connected to the power supply through a control main board. When the electric toothbrush is turn on, the light board and light bead can be controlled by the control main board to operate, which can be applied according to a user's usage need.

The light bead can be implemented as multiple therapeutic equivalents, such as red light therapy, blue light whitening or violet light sterilization, etc. In combination with the brush head, a comprehensive effect of protecting gums and cleaning teeth is achieved.

The power-consuming component can be implemented as a sensor or a photographic component. The internal environment and conditions of the oral cavity can be visually known via the sensor and the photographic element. It can even provide a direct understanding of conditions such as gum inflammation and tooth decay.

### BRIEF DESCRIPTION OF DRAWINGS

FIG. 1 shows a schematic diagram of an electric toothbrush head in an assembled state in the present disclosure.
FIG. 2 shows an exploded structural diagram of an brush head body and a motor of an electric toothbrush head in the present disclosure.
FIG. 3 shows an exploded structural diagram of an electric toothbrush head in the present disclosure.
FIG. 4 shows a diagram of a brush head handle, a motor shaft, and a shaft linkage member that are disassembled from each other and that are in an assembled state itself respectively in the present disclosure.
FIG. 5 shows an illustrative diagram of a conductive circuit of a motor shaft in the present disclosure.
FIG. 6 shows an illustrative diagram of conductive circuits of a first electrical connector and a second electrical connector in the present disclosure.
FIG. 7 shows a front view of an electric toothbrush in the present disclosure.
FIG. 8 shows a section view taken along line A-A in FIG. 7.
FIG. 9 shows an enlarged view at portion B in FIG. 8.

**Reference numerals:**

| | |
|---|---|
| brush head body | 1 |
| bristle assembly | 11 |
| bristle seat | 111 |
| bristle tuft | 112 |
| brush head handle | 12 |
| first insertion cavity | 120 |
| first circumferential limiting rib | 121 |
| first axial limiting groove | 122 |
| shaft linkage member | 2 |
| second insertion cavity | 20 |
| first mounting port | 21 |
| guide slot | 210 |
| second mounting port | 22 |
| first circumferential limiting groove | 23 |
| second axial limiting snap-fit | 24 |
| first axial limiting rib | 25 |
| electrode | 31 |
| insulating mounting base | 32 |
| second wire | 33 |
| motor | 4 |
| motor shaft | 41 |
| second circumferential limiting step surface | 410 |
| shaft connection hole | 411 |
| second axial limiting slot | 412 |
| power-consuming component | 5 |
| light bead | 51 |
| light board | 52 |
| first electrical connector | 61 |
| first wire | 610 |
| elastic deformation portion | 611 |
| second electrical connector | 62 |
| control main board | 7 |
| battery | 8 |

### DESCRIPTION OF EMBODIMENTS

The preferred embodiments described below are only for illustration purposes. Those skilled in the art can conceive of other obvious variations. The basic principles of the present disclosure defined in the following description can be applied to other embodiments, variant technical solutions, improved technical solutions, equivalent technical solutions, and other technical solutions that do not deviate from the spirit and scope of the present disclosure.

Unless explicitly required by the context, similar words such as "include" and "comprise" in the entire specification and claims shall be interpreted as inclusive rather than exclusive or exhaustive. That is to say, it means "including but not limited to". As for the words "and/or" contained in the text, they are used to simplify the expression. For example, "A and/or B" includes both "A and B" and "A or B". The expression "A or B" is interpreted as "A" or "B". The expression "A and B" is interpreted as the simultaneous selection of "A" and "B".

An sonic electric motor or a sonic motor of an electric toothbrush, when supplied with an alternating forward and reverse current at a specific frequency, causes an output shaft of the motor to swing back and forth according to the frequency, so as to truly realize the action of "brushing teeth". The vibration is caused by the attraction and repulsion between a stator and a rotor inside the motor. There is no mechanical friction inside the motor, so that the motor has a higher stability and a large output power, and thus can generate a high-frequency vibration and achieve an effect that is similar to "sound wave". Based on above, the sonic motor is accepted and widely used by people in this field. The above content is necessary for understanding of the present application.

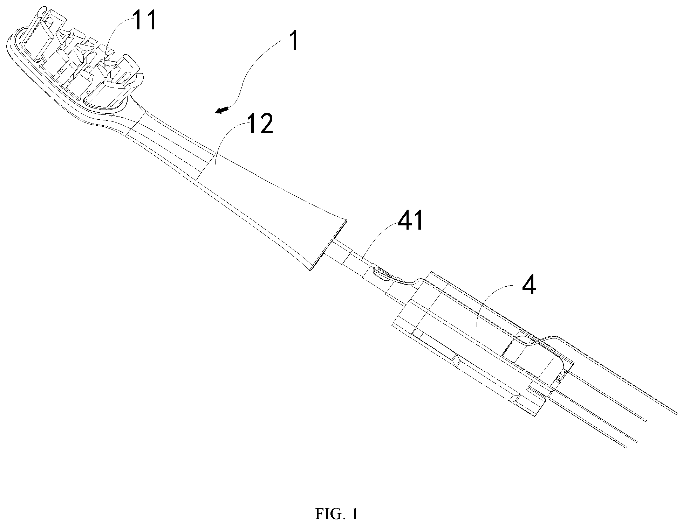

Referring to FIGS. 1 to 6, this embodiment provides an electric toothbrush head, including a brush head body 1, an power-consuming component 5, and a shaft linkage member 2. The power-consuming component 5 is disposed within the brush head body 1. The shaft linkage member 2 is disposed within the brush head body 1 and is configured to be in linkage connection with a motor shaft 41 of a motor 4 in the electric toothbrush. The shaft linkage member 2 is provided with a first electrical connector 61 and a second electrical connector 62. The first electrical connector 61 has one end that is conductively connected to the power-consuming component 5, and another end that is conductively connected to a power supply component of the electric toothbrush. The second electrical connector 62 has one end that is conductively connected to the power-consuming component 5, and another end that is conductively connected to the power supply component of the electric toothbrush. In this embodiment, the first electrical connector 61 and the second electrical connector 62 are arranged on the shaft linkage member 2. In the structure aspect, the shaft linkage member 2 is installed and connected to the brush head body 1; and in the circuit aspect, the two electrical connectors are conductively connected to a positive electrode and a negative electrode of the power-consuming component 5 inside the brush head body 1. In this way, it achieves stable assembly in the structure aspect and convenient electrical conduction in the circuit aspect. The two electrical connectors are stably connected, through the shaft linkage member 2, to the positive electrode and the negative electrode of the power-consuming component 5 on the one hand, and are conveniently conductively connected to a positive electrode and a negative electrode of the power supply component on the other hand. The overall structure uses the shaft linkage member 2 as a carrier, and there is no need for extra structural design, therefore no damage is made to the layout of the components of the electric toothbrush, and the original space occupancy rate of the brush head and the motor shaft 41 will not be reduced.

The above content is the necessary foundation for implementing this embodiment. A further detailed description will be provided with reference to the drawings.

In this embodiment, the sonic motor 4 includes a casing, a stator, a rotor, and bearing components in the openings at both ends of the casing, etc. This is a principle structure of the sonic motor 4 or a sonic electric motor. In this embodiment, the motor shaft 41 of the motor 4 is only processed into a hollow structure, so that the motor shaft 41 forms a wiring channel, a circuit is designed based on the conduction characteristics of the motor shaft 41. This embodiment does not make technical improvements on the principle structure, and thus details of the principle structure are omitted here.

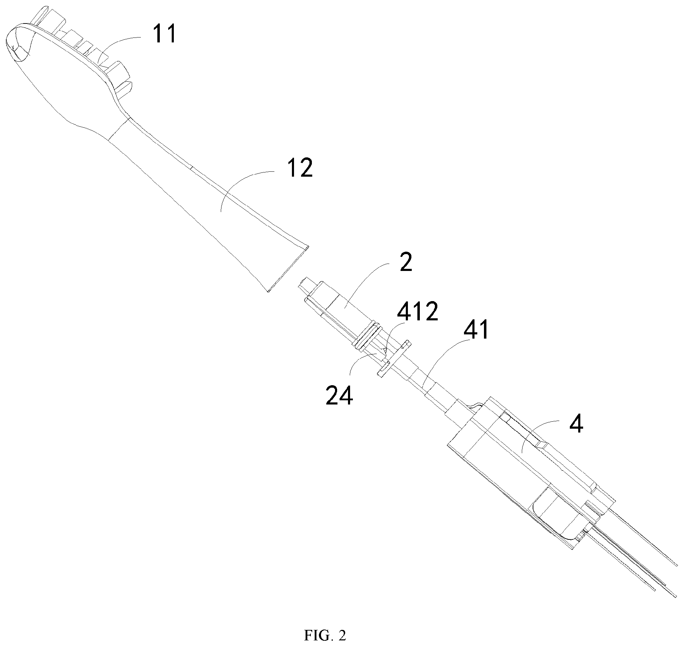

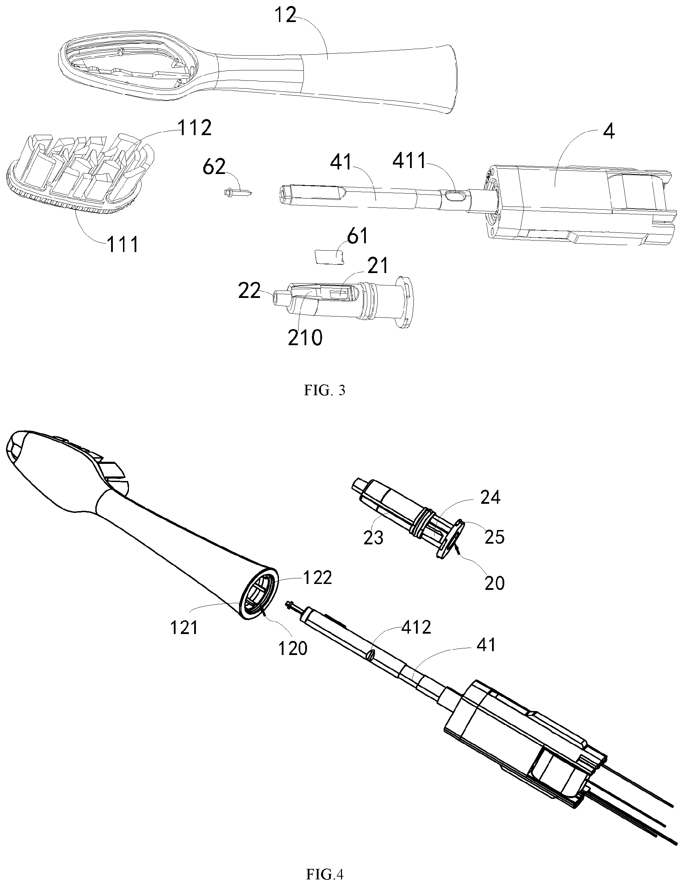

With reference to FIG. 4 and in combination with FIGS. 1 and 2, the brush head body 1 serves as a brushing component of the electric toothbrush, and is linked with the sonic motor 4 of the electric toothbrush through the shaft linkage member 2, so as to swing with a certain amplitude at a high frequency. In the structure aspect, the brush head body 1 includes a bristle assembly 11 and a brush head handle 12. The bristle assembly 11 is mounted to the brush head handle 12, and the power-consuming component 5 is positioned at a location corresponding to the bristle assembly 11. The brush head handle 12 has a first insertion cavity 120, the shaft linkage member 2 is configured to be inserted into the first insertion cavity and to be linked to the brush head handle. The shaft linkage member 2 has a second insertion cavity 20, the motor shaft 41 is configured to be inserted into the second insertion cavity 20 and to be linked to the shaft linkage member 2.

In terms of structural assembly, the shaft linkage member 2 is inserted into the first insertion cavity 120 to achieve linkage assembly with the brush head handle 12. Specifically, the shaft linkage member 2 and the brush head handle 12 are circumferentially fixed relative to each other to achieve synchronous reciprocating rotation. The shaft linkage member 2 and the brush head handle 12 are axially fixed relative to each other to achieve assembly and installation.

To this end, the shaft linkage member 2 is circumferentially restrained relative to the brush head handle 12 through a first circumferential limiting mechanism, and the shaft linkage member 2 is axially restrained relative to the brush head handle 12 through a first axial limiting mechanism.

Specifically, referring to FIG. 4, the first circumferential limiting mechanism includes a first circumferential limiting rib 121 and a first circumferential limiting groove 23. The first circumferential limiting rib 121 and the first circumferential limiting groove 23 are respectively disposed on an inner wall of the first insertion cavity 120 and an outer wall of the shaft linkage member 2. That is, one of the first circumferential limiting rib 121 and the first circumferential limiting groove 23 is disposed on the inner wall of the first insertion cavity 120, the other of the first circumferential limiting rib 121 and the first circumferential limiting groove 23 is correspondingly disposed on the outer wall of the shaft linkage member 2. In this embodiment, the first circumferential limiting rib 121 and the first circumferential limiting groove 122 are arranged along the insertion direction, thereby achieving relative axial position limiting on the one hand and guiding the insertion on the other hand.

Further, referring to FIG. 4, the first axial limiting mechanism includes a first axial limiting rib 25 and a first axial limiting groove 122. The first axial limiting rib 25 and the first axial limiting groove 122 are respectively disposed on an outer wall of the shaft linkage member 2 and an inner wall of the first insertion cavity 120. Similarly to the above, One of the first axial limiting rib 25 and the first axial limiting groove 122 are disposed on the inner wall of the first insertion cavity 120, and the other of the first axial limiting rib 25 and the first axial limiting groove 122 is correspondingly disposed on the outer wall of the shaft linkage member 2. Specifically, the first axial limiting groove 122 is a first ring groove, the first ring groove is disposed on the inner wall of the first insertion cavity 120 that is close to an opening. The first axial limiting rib 25 is a first ring rib, the first ring rib is disposed on the outer wall of the shaft linkage member 2 and is capable of being interference-fitted into the first ring groove. Thus, after the shaft linkage member 2 is inserted into the first insertion cavity 120, the first ring rib is interference-fitted into the first ring groove, thereby achieving assembly and fixation in the insertion direction. Preferably, the first ring rib may be in a one-part form or a multiple-part form.

In the above, the position of the shaft linkage member 2 is restrained in both the axial direction and the circumferential direction, the brush head handle 12 and the shaft linkage member 2 are stably assembled with each other, so as to achieve synchronization of movements without delay and looseness.

Similarly, in terms of structural assembly, the motor shaft 41 is inserted into the second insertion cavity 20 of the shaft linkage member 2, achieving assembly with the shaft linkage member 2 in a linkage manner. Specifically, the motor shaft 41 and the shaft linkage member 2 are fixed relative to each other in the circumferential direction to achieve synchronous reciprocating rotation, and are fixed relative to each other in the axial direction to achieve assembly and installation.

To this end, the motor shaft 41 is inserted into the second insertion cavity 20, the motor shaft 41 is circumferentially restrained relative to the shaft linkage member 2 through a second circumferential limiting mechanism, and the motor shaft is axially restrained relative to the shaft linkage member 2 through a second axial limiting mechanism.

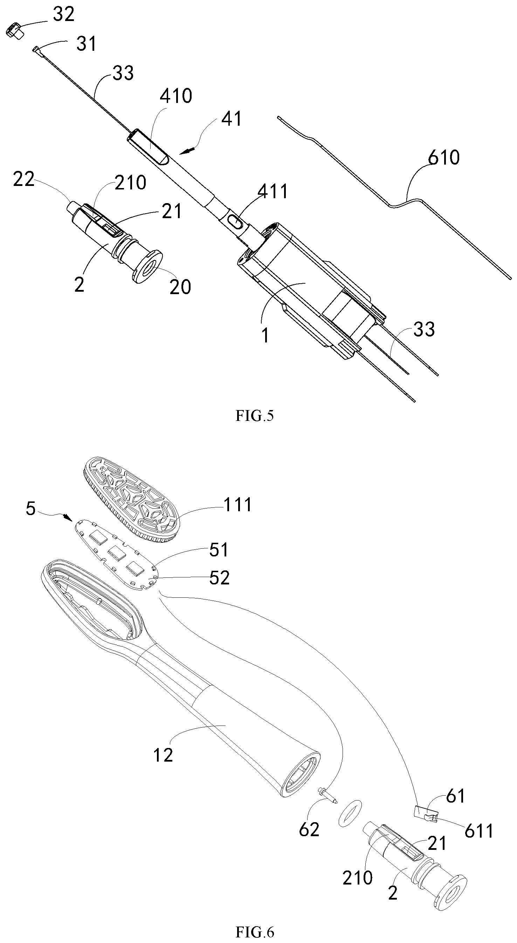

Specifically, referring to FIG. 5, the second circumferential limiting mechanism includes a second circumferential limiting step surface 410 and a second circumferential limiting rib. The second circumferential limiting step surface 410 is formed on the motor shaft 41, and the second circumferential limiting rib is located on the inner wall of the second insertion cavity 20, the second circumferential limiting rib presses against the second circumferential limiting step surface 410 so as to restrict a relative circumferential rotation. Further, the second circumferential limiting step surface 410 is formed on the motor shaft 41 along the insertion direction, the second circumferential limiting rib is formed on the inner wall of the second insertion cavity 20 along the insertion direction, and the second circumferential limiting step surface 410 and the second circumferential limiting rib press against and contact with each other to achieve a synchronous motion in the circumferential direction. It should be noted that although this embodiment does not provide a reference numeral for the second circumferential limiting rib in the drawings, the position and structure of the second circumferential limiting rib can be unambiguously understood by a person skilled in the art based on the above description. In addition, the second circumferential limiting rib may be integral with the shaft linkage member 2, and is located on a surface that is opposite to a guide slot 210on the shaft linkage member 2. The guide slot 210 will be described in conjunction with a first mounting port 21 in detail below.

Further, referring to FIG. 4, the second axial limiting mechanism includes a second axial limiting slot 412 and a second axial limiting snap-fit 24. The second axial limiting slot 412 is formed on the motor shaft 41, and the second axial limiting snap-fit 24 is disposed on the shaft linkage member 2 so as to achieve an axial position limiting relative to the second axial limiting slot 412. Preferably, the second axial limiting snap-fit 24 is an elastic snap-fit. When the motor shaft 41 is inserted into the second insertion cavity 20, the elastic snap-fit snaps into the second axial limiting slot 412 to realize axial position limitation, so as to realize assembly and installation.

In the above, the shaft linkage member 2 achieves stable assembly with the motor shaft 41 by means of position limiting in both the axial direction and the circumferential direction, so as to achieve synchronization of movements without delay and looseness.

The shaft linkage member 2 further serves as a structural carrier for the conductive circuit design, and cooperates with the motor shaft 41, the first electrical connector 61, and the second electrical connector 62 to complete the power supplying to the power-consuming component 5 by the power supply component .

As an implementation of the present embodiment, the one end of the first electrical connector 61 and the one end of the second electrical connector 62 are respectively connected to a positive electrode and a negative electrode of the power-consuming component 5 through wires. The other end of the first electrical connector 61 and the other end of the second electrical connector 62 are respectively connected to the positive electrode and the negative electrode of the power supply component through wires. Specifically, wiring grooves may be provided on the outer wall of the shaft linkage member 2 to route the wires of the first electrical connector 61 and the second electrical connector 62, which does not affect the structure and spatial occupancy of the shaft linkage member 2 and has no effect on the overall structure.

As a preferred implementation of the present embodiment, refer to FIG. 5, the shaft linkage member 2 has a first mounting port 21 at a side thereof, and a second mounting port 22 at a top thereof. The motor 4 is a sonic motor, and the motor shaft 41 is a hollow conductive motor shaft. The hollow conductive motor shaft is provided with a first electrical terminal at a position corresponding to the first mounting port 21. In this embodiment, the first electrical terminal is implemented as an outer surface of the motor shaft 41 corresponding to the first mounting port 21.

In combination with FIGS. 3 and 5, the hollow conductive motor shaft 41 is provided with a second electrical terminal (implemented as an electrode 31 below) at a position corresponding to the second mounting port 22. The first electrical terminal and the second electrical terminal are insulated from each other. The first electrical connector 61 is mounted at the first mounting port 21 and is in electrical contact with the first electrical terminal. The first electrical terminal is connected to the power supply component via a first wire 610. The second electrical connector 62 is mounted at the second mounting port 22 and is in electrical contact with the second electrical terminal. The second electrical terminal is connected to the power supply component via a second wire 33 passing through a hollow inner cavity of the hollow conductive motor shaft. In this implementation, mounting ports are only provided on the top portion and the side portion of the shaft linkage member 2, thereby facilitating the assembly of the first electrical connector 61 and the second electrical connector 62. The wire routing is completed after wires passing through the hollow cavity of the motor shaft 41.

Referring to FIG. 5, in this embodiment, a conductive assembly is included. The conductive assembly includes an electrode 31 and an insulating mounting base 32. The electrode 31 is defined as the second electrical terminal as described above, and the electrode 31 is mounted at the second mounting port 22 via the insulating mounting base 32. One end of the second wire 33 is connected to the electrode 31, and another end of the second wire passes through the hollow conductive motor shaft 41 and is connected to the power supply component. The first electrical terminal is implemented as the outer wall of the motor shaft 41, and the first electrical terminal and the second electrical terminal are insulated from each other by the insulating mounting base 32 to avoid short circuiting therebetween.

The first electrical connector 61 and the second electrical connector 62 are provided to facilitate electrical connection with the first electrical terminal and the second electrical terminal. In a preferred implementation of this embodiment, the second electrical connector 62 is a conductive probe that is inserted into the second mounting port 22 and is in electrical contact with the electrode 31. Further, as shown in FIG. 6, the first electrical connector 61 is a conductive elastic plate that has an elastic deformation portion 611 at its bottom. The shaft linkage member 2 is provided with a guide slot 210 along the insertion direction, and the first mounting port 21 is located in the guide slot 210. The conductive elastic plate compresses the elastic deformation portion 611 so as to be inserted along the guide slot 210 into the first mounting port 21. The elastic deformation portion 611 extends into the first mounting port 21 and is in electrical contact with the first electrical terminal. Preferably, the conductive elastic plate is implemented as a deformable flat spring, and the deformable portion is corresponding to the elastic deformation portion 611. The first electrical terminal is implemented as a partial region of the second circumferential limiting step surface 410, and is in sufficient contact with the deformable part of the flat spring via its flat structure. Further, as shown in FIG. 5, a shaft connection hole 411 is provided on the motor shaft 41 to assist the connection of the first wire 610. One end of the first wire 610 is connected to the shaft connection hole 411, and the motor shaft 41 with a metal material allows the first wire 610 to be conductive connection with the first electrical terminal.

Referring to FIG. 6, the power-consuming component 5 is disposed in the brush head body 1 to enrich the functionality of the electric toothbrush. Specifically, the power-consuming component 5 includes a light board 52 and multiple light beads 51, the multiple light beads are mounted on the light board 52. The light bead 51 is implemented as one of: a blue light whitening bead, a red light physiotherapy bead, or a purple light sterilization bead or any combination thereof. Correspondingly, as shown in FIG. 3, the bristle assembly 11 includes a bristle seat 111 and multiple bristle tufts 112 planted on the bristle seat 111. The bristle tuft 112 is formed by clustering of transparent light-guiding brush filaments, and the bristle tuft 112 is positioned at a location corresponding to the light bead 51, which facilitates the light guidance of the lights of the light bead 51, and is more conducive to the assistive effect on the oral cavity.

Alternatively, the power-consuming component 5 may be implemented as a sensor and/or a photographic component, and facilitates observation and measurement of the environment of the oral cavity. In this embodiment, the power supply component may be implemented as a control main board 7 or a battery 8.

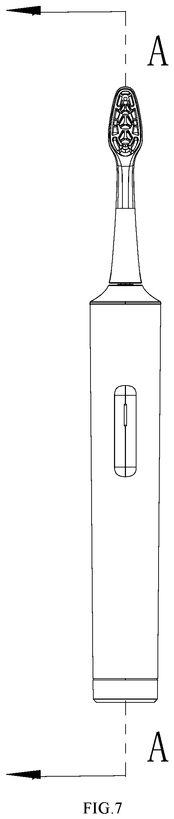

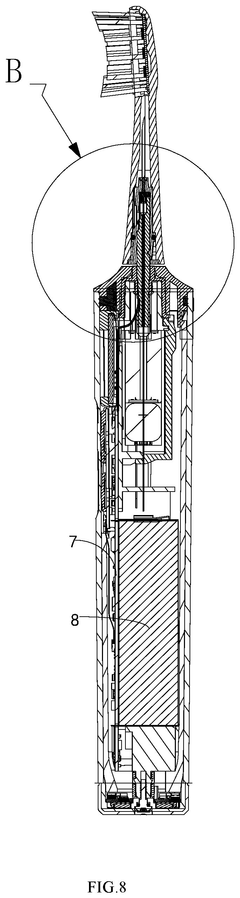

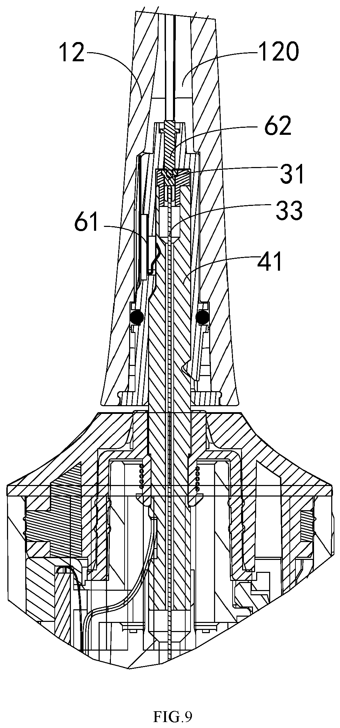

Referring to FIGS. 1 to 6 and in conjunction with FIGS. 7 to 9, the assembling method of the brush head in this embodiment is as follows.

First, the conductive elastic plate and conductive probe are connected to a positive electrode and a negative electrode of the light board 52 via wires. Then, the electrode 31 is mounted within the second mounting port 22 via the insulating mounting base 32, and the conductive elastic plate is inserted along the guide slot 210 and mounted into the first mounting port 21, and the conductive probe is inserted into the second mounting port 22 to be in electrical contact with the electrode 31. Then, One end of the first wire 610 is electrically connected to the shaft connection hole 411 of the motor shaft 41 for later use, and one end of the second wire 33 is connected to the electrode 31 and extends out from the second insertion cavity 20 for later use. Finally, the shaft linkage member 2 is inserted into the first insertion cavity 120 of the brush head handle 12 via the first axial limiting mechanism and the first circumferential limiting mechanism. After the body of the electric toothbrush is mounted, the second wire 33 is routed in the hollow cavity of the motor shaft 41 and extends out of the hollow cavity. At this point, the second wire 33 is insulated from the motor shaft 41. The motor shaft 41 is inserted into the second insertion cavity 20 of the shaft linkage member 2 via the second axial limiting mechanism and the second circumferential limiting mechanism. The other end of the first wire 610 and the other end of the second wire 33 are connected to the control main board 7, and is supplied with power by the battery 8 so as to form a conductive circuit.

It should be noted that the above description when implemented by the skilled person in the field does not have to be reappeared rigidly according to each detailed content. Based on the above description of the overall technical solution described, achieving rapid assembly is enough.

The beneficial effects of the above technical solutions may be resulted from any one of the following items or any combination thereof.

The first electrical connector 61 and the second electrical connector 62 are arranged on the shaft linkage member 2. In the structure aspect, the shaft linkage member 2 is installed and connected to the brush head body 1; and in the circuit aspect, the two electrical connectors are conductively connected to a positive electrode and a negative electrode of the power-consuming component 5 inside the brush head body 1. In this way, it achieves stable assembly in the structure aspect and convenient electrical conduction in the circuit aspect. The two electrical connectors are stably connected, through the shaft linkage member 2, to the positive electrode and the negative electrode of the power-consuming component 5 on the one hand, and are conveniently conductively connected to a positive electrode and a negative electrode of the power supply component on the other hand. The overall structure uses the shaft linkage member 2 as a carrier, and there is no need for extra structural design, therefore no damage is made to the layout of the components of the electric toothbrush, and the original space occupancy rate of the brush head and the motor shaft 41 will not be reduced.

In the present disclosure, the motor shaft 41 of the sonic motor 4 is implemented as a hollow conductive structure. An interior of the motor shaft 41 is implemented as a wiring channel. The conductive assembly passes through and out of the motor shaft 41 along an axis of the motor shaft for connecting to an element, so as to form a first conducting path. The motor shaft 41 itself can be used as a second conducting path due to its conductive property. Both ends of each of the two conducting paths are connected to the power-consuming component 5 and the power supply respectively, so as to form an electrically conductive circuit. The characteristics of the structure of the sonic motor 4 itself is fully utilized. Only the motor shaft 41 needs to be set to be hollow, and there is no need for a significant structural modification to the sonic motor 4. Moreover, the two electrical terminals are integrated with the motor shaft 4 itself, thus the back-and-forth vibrations of the motor 4 at a high frequency does not affect the electrical connection efficiency and no extra wiring layout is required. The two electrical connectors can be contact with corresponding inner parts stably via the shaft linkage member 2 and can be mounted with an external part in an assembled way, and thus has incomparable advantages in both security and convenience.

The external output linkage is completed through the shaft linkage member 2. At the same time, due to a structure of the shaft linkage member 2, the shaft linkage member 2 can be installed on the brush head body and the brush head body 1 can be assembled with the mounting ports on the shaft linkage member 2, so that electrical connection of the two electrical connectors can be achieved.

Wire paths can be completely routed inside the hollow conductive motor shaft 41, or one wire path can be led out from the hollow conductive motor shaft 41 via a wire to connect to an external element. In this way, the present application has a strong adaptability and a wide range of application. It is particularly suitable for a conductive structure in which the power supply is located on one side of the sonic motor 4 and the power-consuming component 5 is located on the other side of the sonic motor 4. It has a strong integration, a high space utilization and low difficulty of arranging wires, and improves the durability.

The two electrical connectors are respectively connected to the two electrical terminals in an assembled way, and the assembling is very convenient. The conducting paths are clear. The conducting path of the power supply component is integrated on the sonic motor 4 and the shaft linkage member 2, and the conducting path of the power-consuming component 5 is integrated into brush head body 1. The conducting path of the power supply component and the conducting path of the power-consuming component 5 only need to be assembled and installed to contact with each other. The shaft linkage member 2 further stabilizes the contact structure between two conducting paths. Compared with the pure wire connection available on the market, reliability in the present application is greatly improved.

The shaft linkage member 2 is inserted into the brush head handle 12 in the electric toothbrush, so that the sonic motor 4 electromagnetically drives the hollow conductive motor shaft 41 and thus the hollow conductive motor shaft 41 drives the brush head handle 12 to rotate reciprocally at a high-frequency through the shaft linkage member 2. Finally, the brush head body 1 is driven to swing at a high frequency. The two electrical connectors are implemented as the conductive probe and conductive elastic plate respectively. The conductive probe is inserted into the first electrode. The conductive elastic plate is assembled with the shaft linkage member 2 via the guide slot 210. The structure of the shaft linkage member 2 achieves stable electrical contact, thereby achieving a high assembly performance and a good stability.

The power-consuming component 5 is implemented as a light board and a light bead with a light effect function, and can be connected to the power supply through a control main board 7. When the electric toothbrush is turn on, the light board and light bead can be controlled by the control main board 7 to operate, which can be applied according to a user's usage need.

The light bead can be implemented as multiple therapeutic equivalents, such as red light therapy, blue light whitening or violet light sterilization, etc. In combination with the brush head, a comprehensive effect of protecting gums and cleaning teeth is achieved.

The power-consuming component 5 can be implemented as a sensor or a photographic component. The internal environment and conditions of the oral cavity can be visually known via the sensor and the photographic element. It can even provide a direct understanding of conditions such as gum inflammation and tooth decay.

The specific embodiments mentioned above do not constitute a limitation on the protection scope of the present disclosure. Those skilled in the art should understand that various modifications, combinations, sub-combinations, and substitutions can be made according to design requirements and other factors. Any modifications, equivalent replacements, and improvements made within the spirit and principles of this disclosure shall be included within the protection scope of the present disclosure.

Those skilled in the art should understand that the embodiments of the present disclosure described above are for illustration only and do not limit the disclosure. The objectives of the present disclosure have been fully and effectively achieved. The functions and structural principles of the present disclosure have been shown and explained in the embodiments. Without departing from these principles, the embodiments of the present disclosure can be modified or varied in any way.

## Claims

1. An electric toothbrush head, comprising:
a brush head body;
a power-consuming component, the power-consuming component being disposed within the brush head body; and
a shaft linkage member, the shaft linkage member being disposed within the brush head body and being configured to be in linkage connection with a motor shaft of a motor in the electric toothbrush; the shaft linkage member being provided with a first electrical connector and a second electrical connector, the first electrical connector having one end that is conductively connected to the power-consuming component and another end that is conductively connected to a power supply component of the electric toothbrush; the second electrical connector having one end that is conductively connected to the power-consuming component and another end that is conductively connected to the power supply component of the electric toothbrush.

2. The electric toothbrush head according to claim 1, wherein the brush head body comprises a bristle assembly and a brush head handle, the bristle assembly is mounted to the brush head handle, and the power-consuming component is positioned at a location corresponding to the bristle assembly; the brush head handle has a first insertion cavity, the shaft linkage member is configured to be inserted into the first insertion cavity and to be linked to the brush head handle; the shaft linkage member has a second insertion cavity, the motor shaft is configured to be inserted into the second insertion cavity and to be linked to the shaft linkage member.

3. The electric toothbrush head according to claim 2, wherein the shaft linkage member is circumferentially restrained relative to the brush head handle through a first circumferential limiting mechanism, and the shaft linkage member is axially restrained relative to the brush head handle through a first axial limiting mechanism.

4. The electric toothbrush head according to claim 3, wherein the first circumferential limiting mechanism comprises a first circumferential limiting rib and a first circumferential limiting groove; the first circumferential limiting rib and the first circumferential limiting groove are respectively disposed on an inner wall of the first insertion cavity and an outer wall of the shaft linkage member.

5. The electric toothbrush head according to claim 3, wherein the first axial limiting mechanism comprises a first axial limiting rib and a first axial limiting groove; the first axial limiting rib and the first axial limiting groove are respectively disposed on an outer wall of the shaft linkage member and an inner wall of the first insertion cavity.

6. The electric toothbrush head according to claim 5, wherein the first axial limiting groove is a first ring groove, the first ring groove is disposed on the inner wall of the first insertion cavity that is close to an opening, the first axial limiting rib is a first ring rib, and the first ring rib is disposed on the outer wall of the shaft linkage member and is capable of being interference-fitted into the first ring groove.

7. The electric toothbrush head according to claim 2, wherein the motor shaft is inserted into the second insertion cavity, the motor shaft is circumferentially restrained relative to the shaft linkage member through the second circumferential limiting mechanism, and the motor shaft is axially restrained relative to the shaft linkage member through the second axial limiting mechanism.

8. The electric toothbrush head according to claim 7, wherein the second circumferential limiting mechanism comprises a second circumferential limiting step surface and a second circumferential limiting rib; the second circumferential limiting step surface is formed on the motor shaft, the second circumferential limiting rib is disposed on an inner wall of the second insertion cavity, and the second circumferential limiting rib presses against the second circumferential limiting step surface so as to restrict a relative circumferential rotation.

9. The electric toothbrush head according to claim 7, wherein the second axial limiting mechanism comprises a second axial limiting slot and a second axial limiting snap-fit, the second axial limiting slot is formed on the motor shaft, and the second axial limiting snap-fit is disposed on the shaft linkage member so as to achieve an axial position limiting relative to the second axial limiting slot.

10. The electric toothbrush head according to claim 1 or 2, wherein the one end of the first electrical connector and the one end of the second electrical connector are respectively connected to a positive electrode and a negative electrode of the power-consuming component via wires; the other end of the first electrical connector and the other end of the second electrical connector are respectively connected to a positive electrode and a negative electrode of the power supply component via wires.

11. The electric toothbrush head according to claim 2, wherein the shaft linkage member has a first mounting port at a side thereof, and has a second mounting port at a top thereof; the motor is a sonic motor, and the motor shaft is a hollow conductive motor shaft; the hollow conductive motor shaft is provided with a first electrical terminal at a position corresponding to the first mounting port and a second electrical terminal at a position corresponding to the second mounting port, the first electrical terminal and the second electrical terminal are insulated from each other; the first electrical connector is mounted at the first mounting port and is in electrical contact with the first electrical terminal, the first electrical terminal is connected to the power supply component via a first wire; the second electrical connector is mounted at the second mounting port and is in electrical contact with the second electrical terminal, the second electrical terminal is connected to the power supply component via a second wire passing through a hollow inner cavity of the hollow conductive motor shaft.

12. The electric toothbrush head according to claim 11, further comprising a conductive assembly, the conductive assembly comprises an electrode and an insulating mounting base, the electrode is defined as the second electrical terminal; the electrode is mounted at the second mounting port via the insulating mounting base, one end of the second wire is connected to the electrode, and another end of the second wire passes through the hollow conductive motor shaft and is connected to the power supply component.

13. The electric toothbrush head according to claim 12, wherein the second electrical connector is a conductive probe, the conductive probe is inserted into the second mounting port and is in electrical contact with the electrode.

14. The electric toothbrush head according to claim 11, wherein the first electrical connector is a conductive elastic plate, the conductive elastic plate has an elastic deformation portion at a bottom thereof; the shaft linkage member is provided with a guide slot along an insertion direction, the first mounting port is located in the guide slot, the first electrical terminal is defined as an outer surface of the motor shaft at a position corresponding to the first mounting port; the conductive elastic plate compresses the elastic deformation portion so as to be inserted along the guide slot into the first mounting port, the elastic deformation portion extends into the first mounting port and is in electrical contact with the first electrical terminal.

15. The electric toothbrush head according to claim 2, wherein the power-consuming component comprises a light board and multiple light beads, the multiple light beads are mounted on the light board; the light bead is implemented as one of a blue light whitening bead, a red light physiotherapy bead, or a purple light sterilization bead or any combination thereof.

16. The electric toothbrush head according to claim 15, wherein the bristle assembly comprises a bristle seat and multiple bristle tufts planted on the bristle seat, the bristle tuft is formed by clustering transparent light-guiding brush filaments, and the bristle tuft is positioned at a location corresponding to the light bead.

17. The electric toothbrush according to claim 2, wherein the power-consuming component is implemented as a sensor and/or a photographic component.
